(19) Europäisches Patentamt
European Patent Office
Office européen des brevets

(11) **EP 4 458 966 A1**

(12) **DEMANDE DE BREVET EUROPEEN**

(43) Date de publication:
**06.11.2024 Bulletin 2024/45**

(21) Numéro de dépôt: **24174116.4**

(22) Date de dépôt: **03.05.2024**

(51) Classification Internationale des Brevets (IPC):
***C12N 15/10*** (2006.01)      ***C12Q 1/6895*** (2018.01)

(52) Classification Coopérative des Brevets (CPC):
**C12Q 1/6895; C12N 15/1003; C12N 15/1017**

(84) Etats contractants désignés:
**AL AT BE BG CH CY CZ DE DK EE ES FI FR GB GR HR HU IE IS IT LI LT LU LV MC ME MK MT NL NO PL PT RO RS SE SI SK SM TR**
Etats d'extension désignés:
**BA**
Etats de validation désignés:
**GE KH MA MD TN**

(30) Priorité:  **05.05.2023  FR 2304497**

(71) Demandeurs:
• **SUEZ International**
**92040 Paris La Défense (FR)**
• **INSTITUT FRANCAIS DE RECHERCHE POUR L'EXPLOITATION DE LA MER - IFREMER**
**29280 Plouzané (FR)**

(72) Inventeurs:
• **PETRAU, Agnès**
**64210 BIDART (FR)**
• **ITOIZ, Sarah**
**64210 BIDART (FR)**
• **SIANO, Raffaele**
**29280 PLOUZANE (FR)**
• **SERGHINE, Joelle**
**29280 PLOUZANE (FR)**

(74) Mandataire: **Lavoix**
**2, place d'Estienne d'Orves**
**75441 Paris Cedex 09 (FR)**

Remarques:
Le document complet y compris le(s) table(s) de référence et le(s) listage(s) de séquence sont téléchargeables sur le site internet de l'OEB

(54) **METHODE DE DETECTION DE MICROALGUES**

(57) La présente invention concerne une méthode de détection d'au moins un microorganisme, de préférence une microalgue, dans un échantillon, de préférence un échantillon d'eau de mer, comprenant les étapes suivantes : filtration de l'échantillon, extraction, purification et amplification de l'ADN et détermination de la présence et/ou quantification du microorganisme à détecter dans l'échantillon. Le microorganisme est de préférence une microalgue du genre *Ostreopsis,* une microalgue de l'espèce *Ostreopsis* cf. *siamensis* ou une microalgue de l'espèce *Ostreopsis* cf. *ovata.* La présente invention a également pour objet un mélange d'amorces anti-sens approprié pour la détection d'une microalgue du genre *Ostreopsis* et/ou d'une microalgue de l'espèce *Ostreopsis cf. ovata,* un kit comprenant ledit mélange d'amorces anti-sens et leur utilisation pour la détection d'au moins une microalgue du genre *Ostreopsis* et/ou une microalgue de l'espèce *Ostreopsis* cf. *ovata.*

**Description**

**Domaine de l'invention**

La présente invention concerne la détection de microorganismes, en particulier de microalgues, dans des échantillons prélevés dans la nature, par exemple des échantillons d'eau de mer.

**Arrière-plan technologique**

Le littoral basque regroupe un ensemble de plages parmi les plus touristiques de la côte Atlantique européenne qui accueille chaque été plus d'un million de personnes. Il constitue un linéaire de plus de 100 km de côte de part et d'autre de la frontière, majoritairement rocheux et abrite un patrimoine naturel remarquable, notamment du point de vue de la biodiversité.

En 2020 et 2021, la côte basque a été le lieu de nombreux signalements, auprès du centre antipoison, de cas d'irritations respiratoire et cutané par des baigneurs ou des promeneurs ayant fréquenté les plages d'Hendaye à Biarritz durant l'été. Les analyses effectuées ont révélé l'efflorescence d'une microalgue appartenant au genre *Ostreopsis,* connue pour engendrer ce genre de troubles ORL (*Oto Rhino Laryngologie*), notamment en Méditerranée. La présence concomitante de deux espèces *Ostreopsis* cf. *siamensis* et O. cf. *ovata* a été identifiée.

Il est donc essentiel de disposer d'une méthode permettant de détecter efficacement ces microalgues, et plus généralement permettant de détecter des microorganismes susceptibles de présenter une toxicité pour l'homme.

L'article de Penna et al. (2007, Journal of Plankton Research, vol. 29, n°1, 19-38) décrit un test basé sur une PCR qualitative et semi-quantitative pour la détection d'algues nuisibles, afin de pouvoir suivre leur développement dans la mer Méditerranée. Les auteurs décrivent les couples d'amorces utilisés pour détecter ces algues, dont les microalgues du genre *Ostreopsis* et les espèces *Ostreopsis* cf. *ovata* et *Ostreopsis* cf. *siamensis.*

L'article de Drouet el al. (2021, Environmental Microbiology, vol. 23, 4956-4979) s'intéresse à la distribution et expansion potentielle *d'Ostreopsis* cf. *siamensis,* comprenant l'analyse par PCR quantitative (qPCR) à partir de différents types d'échantillons d'eau de mer filtrés. Les amorces et conditions de PCR sont notamment telles que décrites dans Penna et al..

Il existe donc un besoin de disposer d'une méthode rapide de détection permettant de détecter et quantifier efficacement des microorganismes, en particulier des microalgues, dans des échantillons prélevés dans la nature, par exemple dans des échantillons d'eau de mer.

**Description de l'invention**

Les Inventeurs ont mis au point une méthode de détection améliorée de microorganismes, basée d'une part sur l'amélioration du rendement d'extraction de l'ADN présent dans l'échantillon de départ et sur l'utilisation d'un plasmide étalon comprenant une séquence consensus du microorganisme à détecter pour la quantification dudit microorganisme.

En particulier, après filtration de l'échantillon, l'étape de lyse comprend avantageusement de placer le filtre dans un récipient en présence d'une solution de lyse, de sorte que le filtre et le rétentat présent sur le filtre soient au contact de la solution de lyse, sous agitation, puis de préférence de broyer mécaniquement le lysat ainsi obtenu. Un meilleur rendement d'extraction de l'ADN est ainsi obtenu.

S'agissant de la quantification par qPCR, la méthode comprend avantageusement l'utilisation d'une gamme étalon obtenue par amplification de dilutions en série d'un plasmide étalon qui comprend une séquence consensus du microorganisme à détecter.

Par ailleurs, s'agissant de la détection des microalgues du genre *Ostreopsis* ou de l'espèce *Ostreopsis* cf. *ovata,* les Inventeurs ont mis en évidence que le couple d'amorces utilisé dans l'art antérieur pour l'amplification de l'ADN de ces microalgues ne permet pas de détecter correctement l'espèce *Ostreopsis* cf. *ovata.* En conséquence, les méthodes de détection connues sous-estiment la quantité de microalgues du genre *Ostreopsis* ou de l'espèce *Ostreopsis* cf. *ovata* présente dans un échantillon, voire concluent de manière erronée à son absence. Les Inventeurs ont alors mis au point un mélange d'amorces anti-sens original permettant d'améliorer la détection des microalgues du genre *Ostreopsis* et/ou de l'espèce *Ostreopsis* cf. *ovata.*

Un premier objet de l'invention est une méthode de détection d'au moins un microorganisme dans au moins un échantillon, comprenant les étapes suivantes :

> a. filtration dudit échantillon, pour obtenir un rétentat comprenant des microorganismes,
> b. extraction de l'ADN des microorganismes du rétentat, pour obtenir une solution comprenant de l'ADN extrait,
> c. purification de l'ADN de la solution comprenant l'ADN extrait, pour obtenir une solution d'ADN purifié,
> d. amplification de l'ADN de la solution d'ADN purifié avec au moins un couple d'amorces spécifique du microorganisme à détecter, et

e. détermination de la présence et/ou quantification du microorganisme à détecter dans l'échantillon.

Le microorganisme à détecter est de préférence une microalgue du genre *Ostreopsis,* une microalgue de l'espèce *Ostreopsis* cf. *siamensis* ou une microalgue de l'espèce *Ostreopsis* cf. *ovata.*

L'étape b) d'extraction comprend de préférence une étape de lyse chimique, pour obtenir un premier lysat et, optionnellement, une étape de lyse mécanique dudit premier lysat.

L'étape de lyse chimique définie ci-dessus comprend de préférence de placer le filtre dans un récipient en présence d'une solution de lyse, de sorte que le filtre et le rétentat présent sur le filtre soient au contact de la solution de lyse et sous agitation.

L'étape c) de purification comprend de préférence une étape de clarification et une étape de précipitation.

L'étape d) comprend de préférence l'amplification de l'ADN d'au moins deux échantillons de référence, chaque échantillon de référence comprenant une concentration connue d'un plasmide, ledit plasmide comprenant une séquence consensus du microorganisme à détecter.

Lorsque ledit au moins un microorganisme à détecter est une microalgue du genre *Ostreopsis* et/ou une microalgue de l'espèce *Ostreopsis* cf. *ovata,* le couple d'amorces dans l'étape d) comprend de préférence un mélange d'amorces anti-sens de séquence SEQ ID NO: 1.

Un autre objet de l'invention est un mélange d'amorces anti-sens approprié pour la détection d'une microalgue du genre *Ostreopsis* et/ou d'une microalgue de l'espèce *Ostreopsis* cf. *ovata,* caractérisé en ce que les amorces anti-sens dudit mélange comprennent la séquence SEQ ID NO: 1.

Un autre objet de l'invention est un kit approprié pour la détection d'une microalgue du genre *Ostreopsis* et/ou d'une microalgue de l'espèce *Ostreopsis* cf. *ovata,* ledit kit comprenant le mélange d'amorces anti-sens tel que défini ci-dessus.

Un autre objet de l'invention concerne l'utilisation d'un mélange d'amorces anti-sens tel que défini ci-dessus ou d'un kit tel que défini ci-dessus, pour la détection d'au moins une microalgue du genre *Ostreopsis* et/ou d'au moins une microalgue de l'espèce *Ostreopsis* cf. *ovata.*

Un autre objet de l'invention concerne un plasmide approprié pour la quantification d'une microalgue dans un échantillon, ledit plasmide comprenant la séquence consensus SEQ ID NO: 13 ou SEQ ID NO: 14.

Microorganisme

La présente invention permet de détecter la présence d'au moins un microorganisme dans un échantillon.

Le microorganisme peut par exemple être un microorganisme toxique ou à risque de toxicité, notamment pour l'homme, et/ou un microorganisme présentant un risque pour l'environnement.

Le microorganisme est de préférence une microalgue.

Par « microalgue », on désigne ici une algue microscopique.

La microalgue est par exemple une algue du genre *Ostreopsis, Alexandrium, Dinophysis, Gambierdiscus* ou/et *Pseudo-nitzschia.*

La microalgue est de préférence une microalgue du genre *Ostreopsis.*

La microalgue du genre *Ostreopsis* est par exemple de l'espèce *O. siamensis, O. ovata, O. lenticularis, O. heptagona, O. mascarenensis, O. labens, O. belizeana, O. carribeana, O. marina, O. fattorussoi* ou *O. rhodesiae.* La microalgue peut également être une microalgue apparentée à ces espèces, telle que *O.* cf. *siamensis, O.* cf. *ovata* ou encore *O.* cf. *lenticularis.*

Echantillon

L'échantillon est un échantillon susceptible de comprendre le ou les microorganismes à détecter, tels que définis ci-dessus, en particulier au moins une microalgue telle que définie ci-dessus.

L'échantillon est de préférence un échantillon liquide.

Si l'échantillon de départ est un substrat solide, par exemple un substrat solide comprenant un biofilm susceptible de comprendre le ou les microorganismes à détecter, ledit biofilm est de préférence remis en suspension dans un milieu liquide, telle que de l'eau de mer filtrée, pour obtenir un échantillon liquide.

L'échantillon peut être un échantillon prélevé dans la nature, par exemple un échantillon d'eau de mer, un échantillon de macroalgue, un substrat rocheux ou un substrat artificiel (par exemple une grille).

L'échantillon peut également être un échantillon prélevé dans un milieu de culture, par exemple un milieu de culture d'au moins un microorganisme tel que défini ci-dessus, notamment d'au moins une microalgue telle que définie ci-dessus.

L'échantillon est de préférence un échantillon d'eau de mer.

L'échantillon d'eau de mer est par exemple prélevé en sub-surface (en particulier de -20 cm à -30 cm) dans une colonne d'eau profonde de 1 m à 1,5 m. De préférence, le flacon est introduit tête en bas à la profondeur du prélèvement et il est rempli en le tournant sur le côté puis vers le haut, pour éviter toutes contaminations. Sur certaines plages, où la colonne d'eau n'atteint pas 1m, l'échantillon doit être prélevé à une profondeur inférieure.

Le volume d'échantillon d'eau de mer prélevé est de préférence d'au moins 1L.

De préférence, l'échantillon ne subit de traitement avant la mise en oeuvre de la méthode selon l'invention. En particulier, l'échantillon n'est de préférence pas traité avec du lugol acide, ni avec du formaldéhyde.

_Amorces et couples d'amorces_

Le microorganisme à détecter est de préférence détecté par amplification de son ADN, après extraction.

L'amplification de l'ADN est effectuée par toute méthode appropriée bien connue de l'homme du métier, telle que la PCR _(« Polymerase Chain Reaction » ou « réaction en chaîne par polymérase »)_ ou par qPCR (PCR quantitative).

La région d'ADN amplifiée est de préférence une région conservée au sein du genre ou de l'espèce du microorganisme à détecter.

La région d'ADN amplifiée est de préférence une région de l'ADN ribosomique (ADNr).

Un couple d'amorces spécifique du microorganisme à détecter est utilisé pour permettre d'amplifier l'ADN dudit microorganisme, si présent dans l'échantillon, afin de pouvoir détecter sa présence ou non dans l'échantillon de départ.

Un couple d'amorces comprend une amorce sens et une amorce anti-sens.

Un couple d'amorces approprié pour détecter une microalgue du genre _Ostreopsis_ permet, de préférence, d'amplifier la région ou une partie de la région ITS 1 et 5.8 S d'une microalgue du genre _Ostreopsis._

Un couple d'amorces approprié pour détecter une microalgue de l'espèce _Ostreopsis_ cf. _siamensis_ permet, de préférence, d'amplifier les régions ou une partie des régions ITS1 et 5.8S de la microalgue _Ostreopsis_ cf. _siamensis._ L'amorce sens et/ou l'amorce anti-sens sont spécifiques _d'Ostreopsis_ cf. _siamensis._ Dans un mode de réalisation préféré, l'amorce sens est spécifique d'une microalgue de l'espèce _Ostreopsis cf. siamensis_ et l'amorce anti-sens est spécifique d'une microalgue du genre _Ostreopsis._

Un couple d'amorces approprié pour détecter une microalgue de l'espèce _Ostreopsis_ cf. _ovata_ permet, de préférence, d'amplifier les régions ou une partie des régions ITS1 et 5.8S de la microalgue _Ostreopsis_ cf. _ovata._ L'amorce sens et/ou l'amorce anti-sens sont spécifiques _d'Ostreopsis_ cf. _ovata._ Dans un mode de réalisation, l'amorce sens est spécifique d'une microalgue _d'Ostreopsis_ cf. _ovata_ et l'amorce anti-sens est spécifique d'une microalgue du genre _Ostreopsis_ ou de l'espèce _Ostreopsis_ cf. _ovata._

Lorsque la méthode concerne la détection de plusieurs espèces de microorganisme du même genre, il peut être avantageux d'utiliser une amorce commune spécifique du genre desdits microorganismes et une amorce spécifique à l'espèce, afin de diminuer le nombre d'amorces utilisées.

Une amorce selon l'invention comprend de préférence au moins 10 nucléotides, de préférence au moins 15 nucléotides, plus préférentiellement au moins 20 nucléotides et/ou au plus 50 nucléotides, de préférence au plus 40 nucléotides, au plus 30 nucléotides.

Typiquement, une amorce selon l'invention comprend 18 à 28 nucléotides, de préférence 20 à 25 nucléotides.

Un couple d'amorces habituellement utilisé dans la littérature pour détecter une microalgue du genre _Ostreopsis_ comprend l'amorce sens de séquence SEQ ID NO: 4 et l'amorce anti-sens de séquence SEQ ID NO: 5.

Cependant, les Inventeurs ont mis en évidence que l'amorce anti-sens de séquence SEQ ID NO: 5 ne permet pas de détecter correctement _Ostreopsis_ cf. _ovata._ De ce fait, la détection de microalgues du genre _Ostreopsis_ et de microalgues de l'espèce _Ostreopsis_ cf. _ovata_ en utilisant cette amorce peut conclure de manière erronée à leur absence et sous-évalue la quantité de ces microalgues présente dans un échantillon.

Les Inventeurs ont alors mis au point un mélange original d'amorces anti-sens, ledit mélange comprenant des amorces anti-sens intégrant des variantes nucléotidiques permettant de détecter efficacement la microalgue de l'espèce _Ostreopsis_ cf. _ovata,_ et donc de ne plus sous-estimer le nombre de microalgues du genre _Ostreopsis_ présentes dans un échantillon, ni le nombre de microalgues de l'espèce _Ostreopsis_ cf. _ovata._ Les variantes nucléotidiques présentes dans la séquence des amorces dudit mélange permettent avantageusement d'englober l'intégralité des polymorphismes observés sur les séquences _d'Ostreopsis._ cf. _ovata._

La présente invention a ainsi pour objet un mélange d'amorces anti-sens approprié pour détecter une microalgue du genre _Ostreopsis_ ou une microalgue de l'espèce _Ostreopsis_ cf. _ovata,_ caractérisé en ce que les amorces dudit mélange comprennent la séquence CCA**R**GA**RY**ATGCCTACATTCAA (séquence SEQ ID NO: 1), dans laquelle R représente G ou A et Y représente T ou C.

Le mélange d'amorces anti-sens tel que défini ci-dessus comprend de préférence au moins deux amorces anti-sens, plus préférentiellement au moins quatre amorces anti-sens, au moins six amorces anti-sens ou au moins sept amorces anti-sens, sélectionnées dans le groupe consistant en :

- CCA**GG**A**GT**ATGCCTACATTCAA (séquence SEQ ID NO: 5),
- CCA**GG**A**GC**ATGCCTACATTCAA (séquence SEQ ID NO: 6),
- CCA**GG**A**AT**ATGCCTACATTCAA (séquence SEQ ID NO: 7),
- CCA**GG**A**AC**ATGCCTACATTCAA (séquence SEQ ID NO: 8),
- CCA**A**GA**GT**ATGCCTACATTCAA (séquence SEQ ID NO: 9),
- CCA**A**GA**GC**ATGCCTACATTCAA (séquence SEQ ID NO: 10),
- CCA**A**GA**AT**ATGCCTACATTCAA (séquence SEQ ID NO: 11) et
- CCA**A**GA**AC**ATGCCTACATTCAA (séquence SEQ ID NO: 12).

De préférence, le mélange d'amorces anti-sens tel que défini ci-dessus comprend les amorces anti-sens de séquence SEQ ID NO: 5, 6, 7, 8, 9, 10, 11 et 12.

Les amorces anti-sens du mélange sont de préférence présentes en quantité égale dans ledit mélange.

Un exemple de couple d'amorces permettant de détecter une microalgue du genre *Ostreopsis* comprend ainsi l'amorce sens de séquence SEQ ID NO: 4 et une amorce anti-sens de séquence SEQ ID NO: 1.

De préférence, plusieurs couples d'amorces comprenant la même amorce sens sont utilisés pour détecter une microalgue du genre *Ostreopsis.* Ces couples d'amorce comprennent par exemple l'amorce sens de séquence SEQ ID NO: 4 et un mélange d'amorces anti-sens comprenant la séquence SEQ ID NO: 1 tel que défini ci-dessus, de préférence les amorces anti-sens de séquences SEQ ID NO: 5 à 12. Ces couples d'amorces permettent d'amplifier un amplicon de 92 paires de bases.

Un exemple de couple d'amorces permettant de détecter une microalgue de l'espèce *Ostreopsis* cf. *siamensis* comprend l'amorce sens de séquence SEQ ID NO: 2 et l'amorce anti-sens de séquence SEQ ID NO: 5. Ce couple d'amorce permet d'amplifier un amplicon de 223 paires de bases.

Un exemple de couple d'amorces permettant de détecter une microalgue de l'espèce *Ostreopsis* cf. *ovata* comprend l'amorce sens de séquence SEQ ID NO: 3 et une amorce anti-sens de séquence SEQ ID NO: 1. De préférence, plusieurs couples d'amorces comprenant la même amorce sens sont utilisés pour détecter une microalgue de l'espèce *Ostreopsis* cf. *ovata.* Ces couples d'amorce comprennent par exemple l'amorce sens de séquence SEQ ID NO: 3 et un mélange d'amorces anti-sens comprenant la séquence SEQ ID NO: 1 tel que défini ci-dessus, de préférence les amorces anti-sens de séquences SEQ ID NO: 5 à 12. Ces couples d'amorces permettent d'amplifier un amplicon de 210 paires de bases.

Plasmide étalon

La présente invention concerne également un plasmide comprenant une séquence consensus du microorganisme à détecter, servant à réaliser une gamme étalon permettant la quantification dudit microorganisme dans l'échantillon. Ce plasmide est également appelé « plasmide étalon » par la suite.

Si plusieurs microorganismes sont à détecter, la méthode de détection selon l'invention comprend de préférence l'utilisation de plusieurs plasmides étalon, chaque plasmide étalon correspondant à un microorganisme à détecter.

Par « séquence consensus », on désigne ici une séquence nucléotidique comprenant les nucléotides les plus fréquents présents à chaque position. Une séquence consensus d'un genre ou d'une espèce de microorganisme est de préférence obtenue par un alignement de multiples séquences provenant de différents individus de ce genre ou de cette espèce de microorganisme.

La séquence consensus est de préférence une région conservée au sein du genre ou de l'espèce du microorganisme à détecter.

Un avantage du plasmide étalon selon l'invention est que la quantification est stable dans le temps et comparable à des équivalents cellulaires.

La présente invention concerne particulièrement un plasmide comprenant une séquence consensus d'une microalgue, en particulier utilisé pour la quantification de ladite microalgue dans un échantillon.

La présente invention concerne particulièrement un plasmide comprenant une séquence consensus *d'Ostreopsis* cf. *siamensis,* de préférence utilisé pour la quantification de la microalgue *Ostreopsis* ou *Ostreopsis* cf. *siamensis* dans un échantillon.

La présente invention concerne également un plasmide comprenant une séquence consensus *d'Ostreopsis* cf. *ovata,* de préférence utilisé pour la quantification de la microalgue *Ostreopsis* ou *Ostreopsis* cf. *ovata* dans un échantillon.

Par exemple, une séquence consensus de la microalgue *Ostreopsis* cf. *siamensis* comprend ou consiste en la séquence SEQ ID NO: 13.

Par exemple, une séquence consensus de la microalgue *Ostreopsis* cf. *ovata* comprend ou consiste la séquence SEQ ID NO: 14.

Le plasmide dans lequel est insérée la séquence consensus peut être tout plasmide approprié bien connu de l'homme du métier permettant l'insertion de la séquence consensus telle que définie ci-dessus.

Le plasmide tel que défini ci-dessus comprend de préférence une séquence consensus du microorganisme à détecter précédée et succédée de 20 à 50 nucléotides et une région de sélection à un antibiotique, par exemple l'ampicilline.

Le plasmide tel que défini ci-dessus est par exemple le plasmide pEX-A128.

Le plasmide tel que défini ci-dessus peut être utilisé pour établir une gamme étalon, permettant de quantifier le microorganisme présent dans l'échantillon.

L'invention concerne également l'utilisation d'un plasmide tel que défini ci-dessus pour la quantification d'au moins un microorganisme dans un échantillon.

L'invention concerne particulièrement l'utilisation d'un plasmide tel que défini ci-dessus pour établir une gamme étalon pour la quantification d'au moins un microorganisme dans un échantillon.

De préférence, le plasmide comprend la séquence SEQ ID NO: 13 pour la quantification de la microalgue *Ostreopsis* ou *Ostreopsis* cf. *siamensis.*

De préférence, le plasmide comprend la séquence SEQ ID NO: 14 pour la quantification de la microalgue *Ostreopsis* ou *Ostreopsis* cf. *ovata.*

On définit ainsi ici un échantillon de référence. Un échantillon de référence comprend une concentration connue (par exemple en nombre de copies par volume) d'un plasmide comprenant une séquence consensus d'un microorganisme à détecter.

Afin de réaliser une gamme étalon, l'ADN d'au moins deux échantillons de référence (de préférence au moins trois échantillons de référence, au moins quatre échantillons de référence ou au moins cinq échantillons de référence) comprenant des concentrations différentes dudit plasmide est amplifié en même temps que l'ADN du ou des échantillons à analyser.

L'ADN amplifié des échantillons de référence permet ainsi de constituer une gamme étalon.

La gamme étalon comprend par exemple des points correspondant à l'ADN amplifié d'échantillons de référence, lesdits échantillons de référence comprenant, avant amplification, des dilutions en série du plasmide, par exemple des dilutions au 10$^{ème}$ du plasmide.

Le premier point de la gamme étalon peut par exemple correspondre à un échantillon de référence comprenant une concentration de départ (i.e. avant amplification) en plasmide comprise de $10^6$ à $10^7$ copies de plasmides par $\mu$l, par exemple $4.10^6$ copies /$\mu$l.

Un exemple de gamme étalon comprend sept points correspondant à des dilutions au 10$^{ème}$, allant de $4.10^6$ copies/$\mu$l à 4 copies/$\mu$l.

Le nombre de copies du plasmide peut être calculé en utilisant la formule suivante :

$$\text{Nombre de copies} = (A \times 6{,}022.10^{23})/(L \times 10^9 \times 660)$$

dans laquelle A est la masse de plasmide en ng, L est la longueur (en bases) du plasmide comprenant la séquence consensus, $6{,}022.10^{23}$ est le nombre d'Avogadro et 660 est le poids moléculaire moyen d'une paire de base.

## Méthode de détection d'au moins un microorganisme dans au moins un échantillon

La présente invention a également pour objet une méthode de détection d'au moins un microorganisme dans au moins un échantillon, comprenant les étapes suivantes :

 a. filtration dudit échantillon, pour obtenir un rétentat comprenant des microorganismes,
 b. extraction de l'ADN des microorganismes du rétentat, pour obtenir une solution comprenant de l'ADN extrait,
 c. purification de l'ADN de la solution comprenant de l'ADN extrait, pour obtenir une solution d'ADN purifié,
 d. amplification de l'ADN de la solution d'ADN purifié avec au moins un couple d'amorces spécifiques du microorganisme à détecter, et
 e. détermination de la présence et/ou quantification du microorganisme à détecter dans l'échantillon.

La méthode selon l'invention peut comprendre de détecter un ou plusieurs microorganismes dans un ou plusieurs échantillons.

Si l'échantillon peut comprendre ou non le ou les microorganismes à détecter, la méthode telle que définie ci-dessus est une méthode de détection d'au moins un microorganisme susceptible d'être présent dans l'échantillon.

L'échantillon est tel que défini ci-dessus dans la partie « Echantillon ».

L'échantillon est de préférence un échantillon d'eau de mer.

Le microorganisme est notamment tel que défini ci-dessus.

Le microorganisme est de préférence une microalgue telle que définie ci-dessus, telle qu'une microalgue du genre *Ostreopsis,* une microalgue de l'espèce *Ostreopsis* cf. *siamensis* ou une microalgue de l'espèce *Ostreopsis* cf. *ovata.*

La méthode de détection telle que définie ci-dessus concerne par exemple la détection d'une, deux ou trois microalgues sélectionnées dans le groupe consistant *Ostreopsis, Ostreopsis* cf. *siamensis* et *Ostreopsis* cf. *ovata.*

*Etape a)*

L'étape a) comprend la filtration de l'échantillon, pour obtenir un rétentat comprenant des microorganismes.

Les microorganismes du rétentat sont l'ensemble des microorganismes présents dans l'échantillon. Ces microorganismes peuvent comprendre ou non le ou les microorganismes à détecter.

Cette étape de filtration permet de concentrer l'échantillon.

Le filtre est en effet approprié pour retenir les microorganismes présents dans l'échantillon et, optionnellement, l'ADN extracellulaire présent dans l'échantillon.

Le rétentat comprend ainsi les microorganismes présents dans l'échantillon et, optionnellement, l'ADN extracellulaire présent dans l'échantillon.

Le volume d'échantillon filtré est par exemple compris de 0,25 à 1 litre, de préférence de 0,5 à 1 litre. Le volume d'échantillon filtré est par exemple égal à 1 litre.

Le filtre a de préférence des pores d'un diamètre compris de 0,45 $\mu$m à 10 $\mu$m, de préférence de 3 $\mu$m à 10 $\mu$m. Le filtre a par exemple des pores d'un diamètre de 3 $\mu$m.

Le filtre est de préférence un filtre en polycarbonate ou en nylon.

Un filtre en polycarbonate, de préférence avec des pores d'un diamètre de 3 $\mu$m, permet avantageusement de récupérer à la fois les microorganismes et l'ADN extracellulaire présents dans l'échantillon.

Le filtre a de préférence un petit diamètre afin de faciliter l'étape d'extraction de l'ADN, en particulier l'étape de lyse. Le filtre a par exemple un diamètre compris entre 40 mm et 50 mm, par exemple un diamètre de 47 mm.

*Etape b)*

L'étape b) comprend l'extraction de l'ADN des microorganismes du rétentat, pour obtenir une solution comprenant de l'ADN extrait.

L'étape b) d'extraction de l'ADN des microorganismes du rétentat comprend :

- une étape de lyse chimique et, optionnellement enzymatique, notamment au moyen d'une solution de lyse, pour obtenir un premier lysat, et
- optionnellement, une étape de lyse mécanique du premier lysat, par exemple par broyage, pour obtenir un deuxième lysat.

L'étape de lyse chimique et, optionnellement enzymatique, comprend de préférence de placer le filtre dans un récipient en présence d'une solution de lyse, de sorte que le filtre et le rétentat présent sur le filtre soient au contact de la solution de lyse. Le récipient est mis, de préférence sous agitation, de préférence au moyen d'un agitateur vortex, par exemple pendant une minute.

Le fait de placer le filtre lui-même dans la solution de lyse permet d'augmenter le rendement de l'extraction.

Toute solution de lyse bien connue de l'homme du métier pour l'extraction d'ADN peut être utilisée.

Par exemple, la solution de lyse comprend au moins un agent chaotropique, au moins un agent dénaturant et/ou au moins un détergent.

Par exemple, la solution de lyse comprend du chlorure de guanidinium et/ou du SDS (dodécylsulfate de sodium) et/ou du CTAB (bromure de cétyltriméthylammonium).

La solution de lyse peut en outre comprendre au moins une enzyme, par exemple une protéinase K et/ou du lysozyme. Dans ce cas, il s'agit à la fois d'une lyse chimique et enzymatique.

L'étape d'extraction comprend de préférence en outre une étape de lyse mécanique du premier lysat, par exemple par broyage, pour obtenir un deuxième lysat.

L'étape de lyse mécanique, en particulier par broyage, permet également d'augmenter le rendement de l'extraction.

L'étape de lyse mécanique est par exemple effectuée en plaçant le premier lysat dans un récipient en présence de billes, puis en broyant l'ensemble.

Les billes sont par exemple des billes de verre, par exemple d'un diamètre de 0,5 mm.

Le broyage peut par exemple comprendre plusieurs cycles, par exemple 3 cycles de broyage, par exemple d'une durée de 30 secondes, par exemple à environ 8000 g (par exemple à 5500 rpm dans un broyeur Precellys Evolution) entrecoupés d'une pause, par exemple de 30 secondes.

La solution comprenant l'ADN extrait est la solution obtenue à l'issue de l'étape de lyse chimique et, optionnellement enzymatique, i.e. le premier lysat, ou, si une étape de lyse mécanique est réalisée, la solution obtenue à l'issue de l'étape de lyse mécanique, i.e. le deuxième lysat.

Dans un mode de réalisation préféré, l'étape b) d'extraction de l'ADN des microorganismes du rétentat comprend :

- une étape de lyse chimique, au moyen d'une solution de lyse, pour obtenir un premier lysat, et
- une étape de lyse mécanique du premier lysat, de préférence par broyage, pour obtenir un deuxième lysat.

Dans ce mode de réalisation préféré, il n'y a donc pas de lyse enzymatique.

*Etave c)*

L'étape c) comprend la purification de l'ADN de la solution comprenant l'ADN extrait, pour obtenir une solution d'ADN purifié.

L'étape c) de purification permet d'obtenir une solution d'ADN purifié, en particulier qui est exempte de composés inhibiteurs de l'étape d) d'amplification de l'ADN.

L'étape c) de purification comprend de préférence une étape de clarification et une étape de précipitation.

L'étape de clarification comprend de préférence une clarification sur une colonne comprenant une membrane de silice, de la solution comprenant l'ADN extrait obtenue à l'étape b).

Par exemple, la solution comprenant l'ADN extrait est transférée sur une colonne comprenant une membrane de silice et l'éluat de clarification est récupéré.

La solution obtenue après clarification, en particulier l'éluat de clarification, est ensuite transférée sur une nouvelle colonne comprenant une membrane de silice permettant de retenir l'ADN.

La fixation de l'ADN est effectuée en ajoutant un tampon de de liaison à la solution obtenue après clarification, pour obtenir un mélange.

Tout tampon de liaison de l'ADN bien connu de l'homme du métier peut être utilisé.

Par exemple, le tampon de liaison comprend du chlorure de guanidinium, des sels et de l'éthanol.

La colonne permettant de retenir l'ADN est ensuite lavée avec au moins un tampon de lavage.

De préférence, la colonne est lavée avec un premier tampon de lavage, puis est lavée une ou deux fois avec un deuxième tampon de lavage.

Le premier tampon de lavage permet d'éliminer les protéines, les métabolites et autres composés inhibiteurs.

Le premier tampon de lavage comprend par exemple du chlorure de guanidinium et de l'isopropanol.

Le deuxième tampon de lavage permet d'éliminer les derniers contaminants dont les traces d'isopropanol et d'assurer un séchage optimal de la membrane de silice.

Le deuxième tampon de lavage comprend par exemple de l'éthanol.

L'ADN est ensuite élué avec un tampon d'élution.

Tout tampon d'élution de l'ADN bien connu de l'homme du métier peut être utilisé.

Par exemple, le tampon d'élution comprend de l'eau et du trisaminométhane.

Pour l'élution, la colonne est de préférence placée dans un récipient comprenant le tampon d'élution à chaud, par exemple à 65°C, par exemple pendant 5 minutes.

Eventuellement, du tampon d'élution est à nouveau ajouté dans le récipient et le récipient est mis en incubation à chaud, par exemple à 65°C, par exemple pendant 5 minutes.

L'ensemble est centrifugé pour récupérer l'éluat comprenant l'ADN purifié.

Cet éluat comprenant l'ADN purifié est la solution d'ADN purifié.

La solution d'ADN purifié peut éventuellement être conservée à -80°C, avant de réaliser l'étape d).

*Etave d)*

L'étape d) comprend l'amplification de l'ADN de la solution d'ADN purifié avec au moins un couple d'amorces spécifiques du microorganisme à détecter.

L'étape d) permet d'obtenir une solution comprenant de l'ADN amplifié si la solution d'ADN purifié comprend de l'ADN du microorganisme à détecter.

L'amplification de l'ADN peut être effectuée à partir d'un volume de la solution d'ADN purifié et, optionnellement, à partir d'un volume de la solution d'ADN purifié dilué, par exemple au 10$^{\text{ème}}$.

La dilution de la solution d'ADN purifié est particulièrement avantageuse en cas de suspicion de la présence de composés inhibiteurs de l'amplification dans ladite solution d'ADN purifié.

L'amplification est de préférence une PCR ou une qPCR.

Si la méthode de détection concerne la détection de deux microorganismes, l'étape d) comprend l'utilisation d'au moins un couple d'amorces spécifique du premier microorganisme à détecter et d'au moins un couple d'amorces spécifique du deuxième microorganisme à détecter.

Si la méthode de détection concerne la détection d'au moins deux microorganismes, l'étape d) comprend l'utilisation d'au moins un couple d'amorces spécifique de chacun des microorganismes à détecter.

Il est possible d'utiliser une même amorce dans des couples d'amorces destinés à détecter des microorganismes différents. C'est notamment le cas lorsqu'une amorce d'un couple d'amorces est spécifique d'une espèce de microorganisme et l'autre amorce est spécifique du genre de ce microorganisme.

Le couple d'amorces spécifique d'un microorganisme à détecter est par exemple tel que défini ci-dessus dans la section « Amorces et amplifications d'amorces ».

Lorsque le microorganisme est la microalgue *Ostreopsis* ou la microalgue *Ostreopsis* cf. *ovata,* l'étape d) comprend de préférence l'amplification de l'ADN de la solution d'ADN purifié avec une amorce sens spécifique dudit microorganisme et un mélange d'amorces anti-sens, lesdites amorces anti-sens comprenant la séquence SEQ ID NO: 1.

Lorsque le microorganisme à détecter est la microalgue *Ostreopsis,* l'étape d) comprend, de préférence, l'amplification de l'ADN de la solution d'ADN purifié avec une amorce sens spécifique *d'Ostreopsis* et un mélange d'amorces anti-sens, lesdites amorces anti-sens comprenant la séquence SEQ ID NO: 1.

Lorsque le microorganisme est la microalgue *Ostreopsis* cf. *ovata,* l'étape d) comprend, de préférence, l'amplification de l'ADN de la solution d'ADN purifié avec une amorce sens spécifique *d'Ostreopsis* cf. ovata et un mélange d'amorces anti-sens, lesdites amorces anti-sens comprenant la séquence SEQ ID NO: 1.

Le mélange d'amorces anti-sens est notamment tel que défini ci-dessus.

L'étape d'amplification peut être mise en oeuvre en utilisant un kit tel que défini ci-dessous dans la section « kit ».

L'homme du métier sait déterminer les conditions appropriées pour une étape d'amplification d'ADN.

A titre d'exemple, le cycle d'amplification peut comprendre :

- 10 minutes à 95°C pour la phase initiale de dénaturation,
- 40 cycles d'hybridation comprenant 95°C pendant 15 secondes, 60°C pendant 15 secondes, 72°C pendant 15 secondes, et
- 1 minute à 95°C pour la phase finale.

Si l'amplification est une qPCR, l'acquisition de la fluorescence est par exemple effectuée toutes les 5 secondes pendant les cycles d'hybridation. Une courbe de fusion est de préférence réalisée sur une gamme de température allant de 65 à 95 °C, en particulier avec des incrément 0,5°C.

Lorsque l'amplification est une qPCR, l'étape d) comprend de préférence l'amplification d'une gamme étalon d'ADN, pour la quantification.

De manière avantageuse, la gamme étalon d'ADN est constituée de l'ADN d'échantillons de référence tels que définis ci-dessus dans la section « Plasmide étalon ».

L'étape d) comprend ainsi de préférence l'amplification de l'ADN présent dans des échantillons de référence tels que définis ci-dessus.

Dans ce cas, pour chaque microorganisme à détecter, l'ADN d'échantillons de référence comprenant des concentrations connues d'un plasmide comprenant une séquence consensus du microorganisme à détecter sont également amplifiés à l'étape d).

Les échantillons de référence correspondant au microorganisme à détecter permettent d'obtenir une gamme de quantification pour ce microorganisme.

Typiquement, des échantillons de référence comprenant des concentrations connues d'un plasmide comprenant une séquence consensus *d'Ostreopsis* cf. *siamensis* sont utilisés pour établir une gamme étalon pour la quantification *d'Ostreopsis* cf. *siamensis*. La séquence consensus *d'Ostreopsis* cf. *siamensis* par exemple comprend ou consiste en la séquence SEQ ID NO: 13.

De même, des échantillons de référence comprenant des concentrations connues d'un plasmide comprenant une séquence consensus d'*Ostreopsis* cf. *ovata* sont utilisés pour établir une gamme étalon pour la quantification d'*Ostreopsis* cf. *ovata*. La séquence consensus d'*Ostreopsis* cf. *ovata* par exemple comprend ou consiste en la séquence SEQ ID NO: 14.

Par ailleurs, des échantillons de référence comprenant des concentrations connues d'un plasmide comprenant une séquence consensus d'*Ostreopsis* cf. *siamensis* (par exemple comprenant ou consistant en la séquence SEQ ID NO: 13) ou d'*Ostreopsis* cf. *ovata* (par exemple comprenant ou consistant en la séquence SEQ ID NO: 14) peuvent être utilisés pour établir une gamme étalon pour la quantification d'*Ostreopsis*.

*Etave e)*

L'étape e) comprend la détermination de la présence et/ou la quantification du microorganisme à détecter dans l'échantillon.

Si de l'ADN est amplifié à l'issue de l'étape d), en utilisant un couple d'amorces spécifiques du microorganisme à détecter, alors ledit microorganisme est présent dans l'échantillon. La détection de l'ADN amplifié peut être réalisée par électrophorèse, par exemple sur gel d'agarose. La quantification peut par exemple être effectuée lorsque l'amplification à l'étape d) est une qPCR.

Si aucun ADN n'est amplifié à l'issue de l'étape d), en utilisant un couple d'amorces spécifiques du microorganisme à détecter, alors ledit microorganisme n'est pas présent dans l'échantillon.

Lorsque l'étape d) est une qPCR, la quantité ou concentration du microorganisme à détecter dans l'échantillon est déterminée, en particulier à partir de la gamme étalon d'ADN.

Dans un mode de réalisation avantageux, la présente invention a pour objet une méthode de détection telle que défini ci-dessus d'au moins une microalgue, de préférence une microalgue du genre *Ostreopsis,* une microalgue *Ostreopsis* cf. *siamensis* et/ou une microalgue *Ostreopsis* cf. *ovata,* dans au moins un échantillon, de préférence au moins un échantillon d'eau de mer, comprenant les étapes suivantes :

a. filtration dudit échantillon, pour obtenir un rétentat comprenant des microorganismes,
b. extraction de l'ADN des microorganismes du rétentat, pour obtenir une solution comprenant de l'ADN extrait,
c. purification de l'ADN de la solution comprenant de l'ADN extrait, pour obtenir une solution d'ADN purifié,
d. amplification de l'ADN de la solution d'ADN purifié avec au moins un couple d'amorces spécifiques de la microalgue à détecter, et
e. détermination de la présence et/ou quantification de ladite microalgue dans l'échantillon.

Kit

La présente invention a également pour objet un kit approprié la détection d'au moins un microorganisme tel que défini ci-dessus, en particulier approprié pour la mise en oeuvre d'une méthode de détection d'au moins un microorganisme telle que défini ci-dessus.

Le kit tel que défini ci-dessus est de préférence approprié pour la détection d'une microalgue du genre *Ostreopsis* et/ou d'une microalgue *Ostreopsis* cf. *ovata.*

Le kit tel que défini ci-dessus comprend de préférence au moins un couple d'amorces permettant d'amplifier l'ADN (i) de microalgues du genre *Ostreopsis* et/ou de microalgues de l'espèce *Ostreopsis* cf. *ovata.*

De préférence, le kit tel que défini ci-dessus comprend en outre au moins un couple d'amorces permettant d'amplifier l'ADN de microalgues de l'espèce *Ostreopsis cf. siamensis.*

Le kit tel que défini ci-dessus comprend de préférence un mélange d'amorces anti-sens, dans lequel les amorces anti-sens comprennent la séquence SEQ ID NO: 1.

Le kit tel que défini ci-dessus comprend de préférence un mélange d'amorces anti-sens, lesdites amorces comprenant les séquences SEQ ID NO: 5 à 12.

Le kit tel que défini ci-dessus comprend de préférence :

- un mélange d'amorces anti-sens, lesdites amorces comprenant les séquences SEQ ID NO: 5 à 12,
- une amorce sens de séquence SEQ ID NO: 3 et/ou une amorce sens de séquence SEQ ID NO : 4,
- optionnellement, une amorce sens de séquence SEQ ID NO : 2, et
- optionnellement, une amorce anti-sens de séquence SEQ ID NO : 5.

Un kit préféré tel que défini ci-dessus comprend :

- un mélange d'amorces anti-sens, lesdites amorces comprenant les séquences SEQ ID NO: 5 à 12,
- une amorce sens de séquence SEQ ID NO: 3,
- une amorce sens de séquence SEQ ID NO : 4,
- une amorce sens de séquence SEQ ID NO : 2, et
- une amorce anti-sens de séquence SEQ ID NO : 5.

De préférence, le kit tel que défini ci-dessus comprend en outre :

- au moins une enzyme polymérase,
- des dNTPs,

- un tampon
- des ions magnésium et/ou
- un colorant de l'ADN ou de l'ARN.

Le kit tel que défini ci-dessus peut en outre comprendre :

- au moins un filtre, de préférence un filtre en polycarbonate ou en nylon,
- au moins un tampon de lyse,
- au moins un tampon de liaison,
- au moins un tampon de précipitation, et/ou
- au moins un tampon d'élution.

Le filtre, le tampon de lyse, le tampon de liaison, le tampon de précipitation et le tampon d'élution sont notamment tels que définis ci-dessus.

Utilisation d'un mélange d'amorces ou d'un kit

La présente invention a également pour objet l'utilisation d'un mélange d'amorces anti-sens tel que défini ci-dessus, pour la détection d'au moins une microalgue du genre *Ostreopsis* et/ou d'au moins une microalgue de l'espèce *Ostreopsis* cf. *ovata*.
Le mélange d'amorces anti-sens est notamment tel que défini ci-dessus.
Le mélange d'amorces anti-sens comprend de préférence les amorces anti-sens de séquences SEQ ID NO: 5 à 12.
Le mélange d'amorces anti-sens est par exemple utilisé avec l'amorce sens de séquence SEQ ID NO: 4 pour détecter une microalgue du genre *Ostreopsis.*
Le mélange d'amorces anti-sens est par exemple utilisé avec l'amorce sens de séquence SEQ ID NO: 3 pour détecter une microalgue de l'espèce *Ostreopsis* cf. *ovata.*
La présente invention a également pour objet l'utilisation d'un kit tel que défini ci-dessus dans la section « kit », pour la détection d'au moins une microalgue du genre *Ostreopsis* et/ou d'au moins une microalgue de l'espèce *Ostreopsis* cf. *ovata.*
La présente invention a également pour objet l'utilisation telle que définie ci-dessus pour la détection de (i) au moins une microalgue du genre *Ostreopsis* et/ou d'au moins une microalgue de l'espèce *Ostreopsis* cf. *ovata* et (ii) optionnellement, pour la détection d'au moins une microalgue de l'espèce *Ostreopsis* cf. *siamensis.*
D'autres caractéristiques et avantages de l'invention ressortiront mieux des exemples qui suivent, donnés à titre illustratif et non limitatif.

**Listage de séquences**

La séquence SEQ ID NO: 1 correspondant à la séquence des amorces anti-sens spécifiques *d'Ostreopsis* cf. *ovata.*
La séquence SEQ ID NO: 2 correspond à la séquence d'une amorce sens spécifique *d'Ostreopsis* cf. *siamensis.*
La séquence SEQ ID NO: 3 correspond à la séquence d'une amorce sens spécifique *d'Ostreopsis* cf. *ovata.*
La séquence SEQ ID NO: 4 correspond à la séquence d'une amorce sens spécifique d'*Ostreopsis.*
La séquence SEQ ID NO: 5 correspond à la séquence d'une amorce anti-sens spécifique d'*Ostreopsis.*
Les séquences SEQ ID NO: 6 à SEQ ID NO: 12 correspondent à la séquence d'une amorce anti-sens spécifique d'*Ostreopsis* cf. *ovata.*
La séquence SEQ ID NO: 13 correspond à une séquence consensus de la microalgue *Ostreopsis* cf. *siamensis.*
La séquence SEQ ID NO: 14 correspond à une séquence consensus de la microalgue *Ostreopsis* cf. *ovata.*

**Figures**

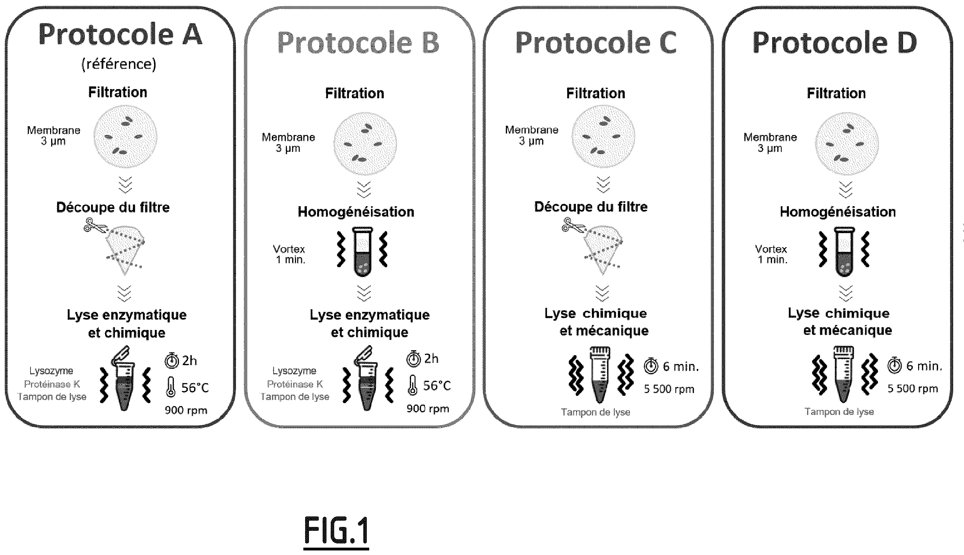

[Fig. 1] La figure 1 décrit les protocoles d'extraction d'ADN testés sur les cultures d'*O.* cf. *siamensis* et d'*O.* cf. *ovata,* où le protocole A est le protocole de référence et les protocoles B, C et D en sont des variantes.

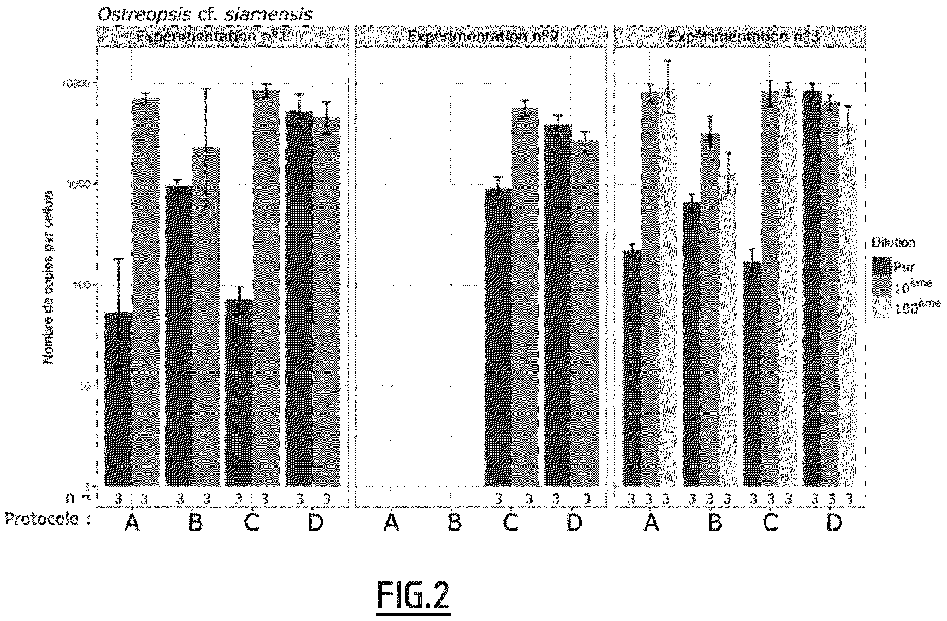

[Fig. 2] La figure 2 décrit les moyennes et écart-types du nombre de copies par cellule d'*O.* cf. *siamensis* sur plusieurs niveaux de dilution extraits avec les protocoles A, B, C et D. Cette analyse renseigne sur les performances d'extraction des protocoles en fonction de la concentration cellulaire d'*O.* cf. *siamensis.* Les trois séries analytiques sont représentées séparément pour évaluer la répétabilité des protocoles. Chaque dilution a été réalisée en triplicat (n). Ct : cycle de seuil ou «Cycle threshold ».

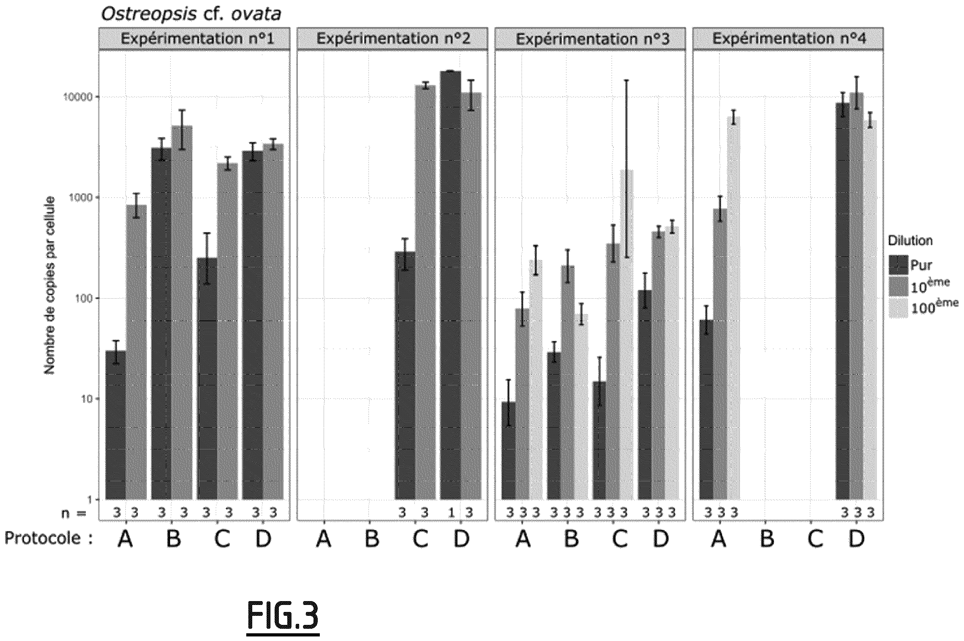

[Fig. 3] La figure 3 décrit les moyennes et écart-types du nombre de copies par cellule d'*O.* cf. *ovata* sur plusieurs niveaux de dilution extraits avec les protocoles A, B, C et D. Cette analyse renseigne sur les performances d'extraction des protocoles en fonction de la concentration cellulaire d'*O.* cf. *ovata.* Les quatre séries analytiques sont représentées séparément pour évaluer la répétabilité des protocoles. Chaque dilution a été réalisée en triplicat (n). Ct : cycle de seuil ou «Cycle threshold ».

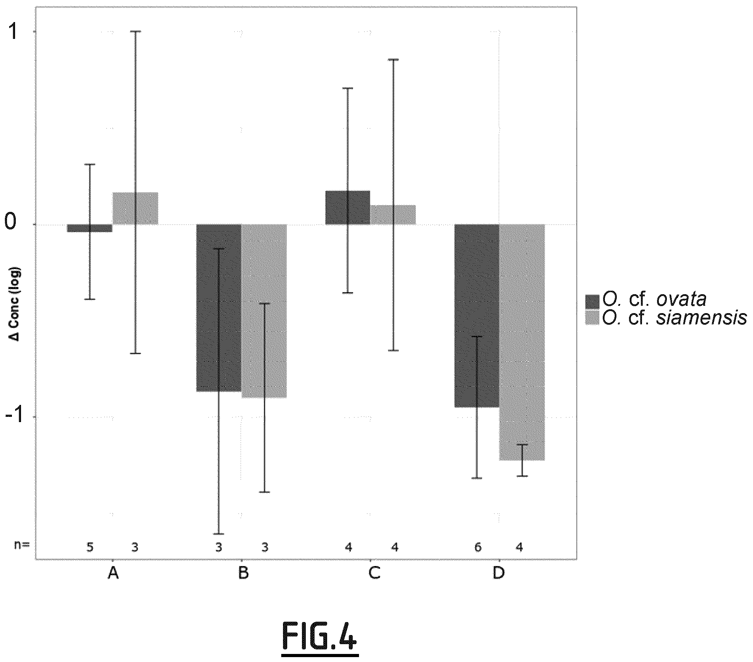

[Fig. 4] La figure 4 décrit les moyennes et écart-types des différences entre deux niveaux de dilution extraits avec les protocoles A, B, C et D sur des cultures d'*O.* cf. *siamensis* et d'*O.* cf. *ovata.* Les différences de concentration sont exprimées en logarithme base 10. Le nombre de séries analytiques (n) est indiqué sur la figure.

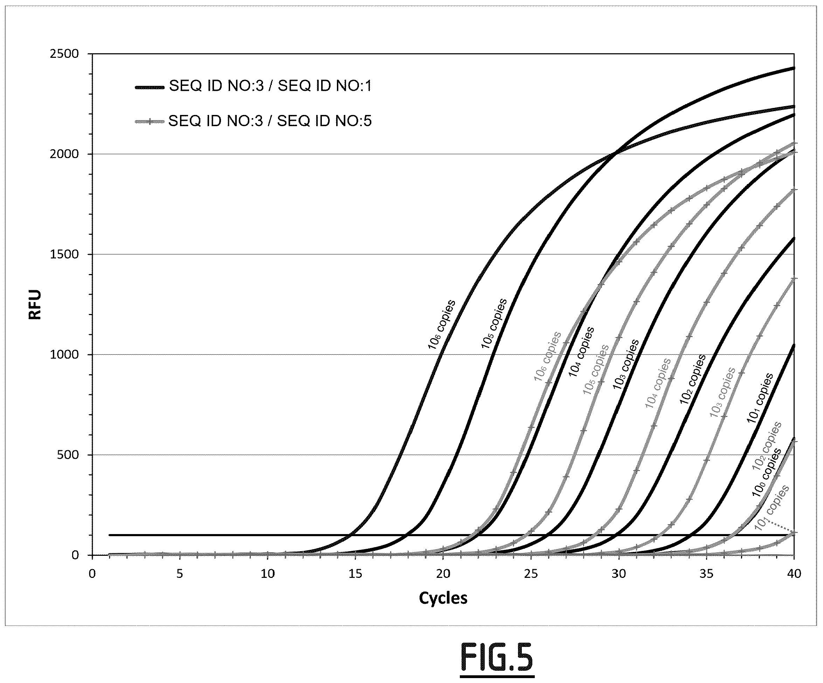

[Fig. 5] La figure 5 décrit les courbes d'amplification d'une gamme d'étalon plasmide *O.* cf. *ovata* (nombre de copies) avec les couples d'amorces SEQ ID NO: 3/ SEQ ID NO: 1 et SEQ ID NO: 3/ SEQ ID NO: 5.

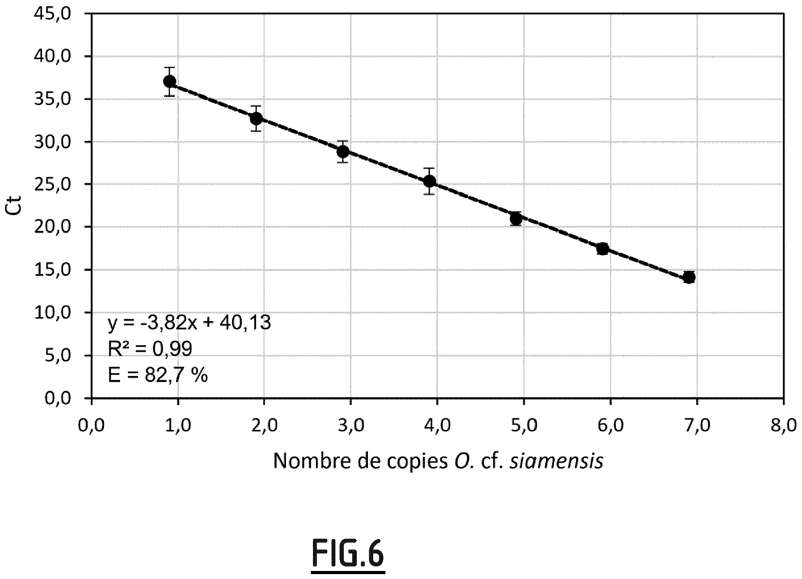

[Fig. 6] La figure 6 décrit la gamme standard pour *O.* cf. *siamensis* (n=12 runs de qPCR) Le nombre de copies par dilution est transformé par un logarithme base 10. Les paramètres de la qPCR sont représentés par l'équation de droite (y), la linéarité ($R^2$) et l'efficacité (E). Ct : cycle de seuil ou «Cycle threshold ». RFU : Unité de fluorescence relative ou « relative fluorescence unit ».

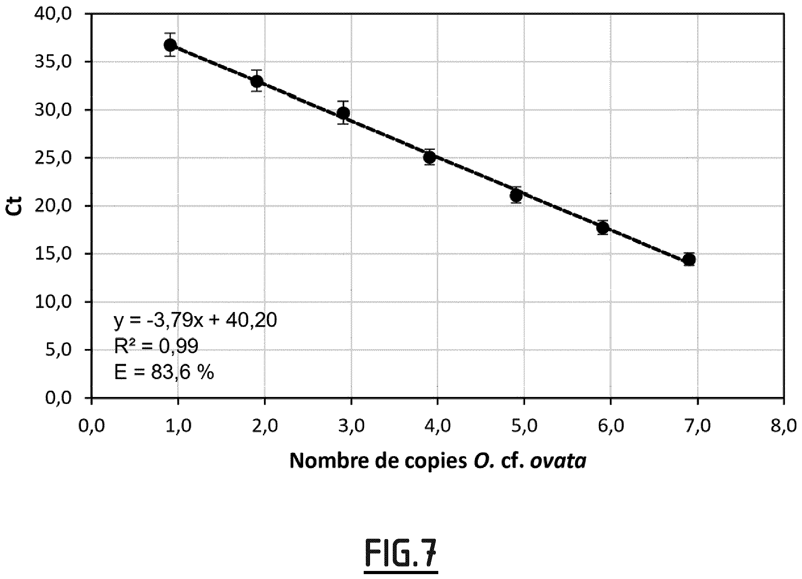

[Fig. 7] La figure 7 décrit la gamme standard pour *O.* cf. *ovata* (n=11 runs de qPCR). Le nombre de copies par dilution est transformé par un logarithme base 10. Les paramètres de la qPCR sont représentés par l'équation de droite (y), la linéarité ($R^2$) et l'efficacité (E). Ct : cycle de seuil ou «Cycle threshold ». RFU : Unité de fluorescence relative ou « relative fluorescence unit »

## Exemples

La méthode déployée a pour objectif de détecter et de quantifier *Ostreopsis* spp., *Ostreopsis* cf. *siamensis* et *Ostreopsis* cf. *ovata* par qPCR à partir d'ADN extraits de cultures ou de matrices environnementales. Le protocole d'extraction d'ADN est réalisé par l'intermédiaire d'un kit commercial, le NucleoSpin Plant II Mini de chez Macherey-Nagel. Brièvement, un volume d'échantillon (de préférence 1 litre) est filtré sur une membrane de 3 μm en polycarbonate de diamètre 47 mm. La membrane est vortexée pendant 1 minute dans un tampon de lyse contenant des agents chaotropiques, des agents dénaturants et un détergent. Le lysat est ensuite broyé en présence de billes de verre d'un diamètre de 0,5 mm, en suivant 3 cycles de broyage d'une durée de 30 secondes à 5500 rpm entrecoupés d'une pause de 30 secondes, dans un broyeur Precellys Evolution. L'ADN contenu dans le lysat est clarifié sur une membrane de silice, puis fixé sur une nouvelle membrane de silice à l'aide d'un tampon de liaison. Il est ensuite purifié grâce à l'utilisation de tampons de lavage contenant des concentrations croissantes en isopropanol et éthanol. L'ADN est finalement élué dans 100 μl de tampon d'élution contenant 5 mM de trisaminométhane. Le protocole de qPCR au SybrGreen est issu de la publication de Drouet *et al.* (2021). Les trois couples d'amorce sur la région ITS-5.8 rDNA, conçus par Penna *et al.* (2007) ou selon l'invention (cf. exemple 2), ont été utilisés pour la quantification d'*Ostreopsis* spp., *O.* cf. *siamensis* and *O.* cf. *ovata.* Les qPCR ont été réalisées sur la base du kit commercial Platinum® SYBR® Green qPCR SuperMix-UDG (Invitrogen) dont le mix réactionnel est composé de 0,4μM de chaque amorce, 1mM de MgCl$_2$, 1X de mix SybrGreen, 2μl d'extrait d'ADN et de l'eau de grade moléculaire. Chaque run de qPCR est réalisé avec une gamme standard (*O.* cf. *siamensis* ou *O.* cf. *ovata,* en utilisant les plasmides étalon selon l'invention dans l'exemple 3) et deux contrôles négatifs (qPCR et extraction). Les extraits d'ADN sont déposés en simplicat. Les conditions du cycle d'amplification sont les suivantes : 10 minutes à 95°C pour la phase initiale de dénaturation, suivi par 40 cycles d'hybridation (95°C pendant 15 secondes, 60°C pendant 15 secondes, 72°C pendant 15 secondes incluant la phase d'acquisition de la fluorescence) et se termine par une phase à 95°C pendant 1 minute. La courbe de fusion est réalisée sur une gamme de température allant de 65 à 95 °C (incrément 0,5°C) dont l'acquisition se fait toutes les 5 secondes.

## Exemple 1: Amélioration de l'étape de lyse

Le protocole de référence et trois de ses variantes ont été testés pour déterminer la meilleure méthode d'extraction d'ADN pour *O.* cf. *siamensis* et *O.* cf. *ovata.* Les modalités des différents protocoles sont représentées en Figure 1, le protocole A étant le protocole de référence développé par l'Ifremer et le protocole D correspondant à la méthode selon l'invention.

Dans le protocole A, la membrane est placée dans le tampon de lyse sans agitation et il s'agit d'une lyse chimique et enzymatique.

Dans le protocole B, la membrane est placée dans le tampon de lyse sous agitation (vortexage) et il s'agit d'une lyse chimique et enzymatique.

Dans le protocole C, la membrane est placée dans le tampon de lyse sans agitation et il s'agit d'une lyse chimique et mécanique (par broyage).

Le protocole D comprend à la fois de placer la membrane dans le tampon de lyse sous agitation (vortexage) pendant une minute et il s'agit d'une lyse chimique et mécanique (par broyage).Les rendements des ADN extraits sont évalués pour chaque condition expérimentale (lyse mécanique et enzymatique, avec ou sans étapes additionnelles) réalisée en triplicat sur des cultures d'*O.* cf. *siamensis* et *O.* cf. *ovata.*

Pour les protocoles A, B et C, le nombre de copies par cellule est hétérogène entre les dilutions. Une baisse de la performance d'extraction est observée avec l'augmentation de la concentration cellulaire (Fig. 2, Fig. 3 et Fig. 4). Concernant le protocole D, le nombre de copies par cellule reste stable qu'importe la dilution et semble reproductible entre les différentes expérimentations menées sur les deux espèces (Fig. 2 et Fig. 3). Ce protocole induit moins de variabilité et permet une extraction fiable des ADN (Fig. 4).

Le protocole D est généralisable à l'extraction d'ADN de tout microorganisme, en particulier de toute microalgue.

**Exemple 2** : **Amélioration de la détection d'*Ostreopsis* et *Ostreopsis* cf. *ovata* en utilisant un mélange d'amorces anti-sens**

Les couples d'amorces utilisés pour chaque microalgue cible à détecter, leur séquence et la taille de l'amplicon obtenu sont indiqués dans le tableau 1.

**[Tableau 1]**

| Nom de l'amorce | Séquence des amorces et sonde (5' - 3') | Cible | Taille Amplicon |
|---|---|---|---|
| Ostreopsis_F | AAAACGATATGAAGAGTGCAGC (SEQ ID NO: 4) | 5.8S *Ostreopsis* spp. | 92 pb |
| Ostreopsis_R _dO | CCARGARYATGCCTACATTCAA R = G ou A; Y = T ou C (SEQ ID NO: 1) | | |
| Siamensis_F | TGTTACCATTGCTGAGTTTG (SEQ ID NO: 2) | ITS1-5.8S O. cf. *siamensis* | 223 pb |
| Ostreopsis_R | CCAGGAGTATGCCTACATTCAA (SEQ ID NO: 5) | | |
| Ovata_F | CAATGCTCATGTCAATGATG (SEQ ID NO: 3) | ITS1-5.8S O. cf. *ovata* | 210 pb |
| Ostreopsis_R _dO | CCARGARYATGCCTACATTCAA R = G ou A ; Y = T ou C (SEQ ID NO: 1) | | |

Les couples d'amorces SEQ ID NO: 3/ SEQ ID NO: 1 et SEQ ID NO: 3/ SEQ ID NO: 5 ont été testés en simultané sur la même gamme de plasmides *O.* cf. *ovata.* Un décalage d'environ 6 Ct, soit 2 log, a été mis en évidence entre SEQ ID NO: 3/ SEQ ID NO: 1 et SEQ ID NO :3/ SEQ ID NO: 5 (cf. Fig. 5). Le couple d'amorces SEQ ID NO:3/ SEQ ID NO:1 permet une détection plus globale des séquences d'*O.* cf. *ovata* et donc une plus grande sensibilité dans la quantification.

**Exemple 3** : **Amélioration de la quantification du microorganisme en utilisant un plasmide étalon**

La séquence consensus de chaque plasmide a été obtenue à partir des séquences du cluster ribosomique 18S-ITS1-5.8S extraites de la banque de données Genbank pour chacune des deux espèces. Ces séquences ont été alignées par ClustalO sur Seaview puis sélectionnées en fonction de leur longueur et de leur qualité. L'alignement pour *O.* cf. *siamensis* comporte 179 séquences et 1 482 caractères ; l'alignement pour *O.* cf. *ovata* comporte 67 séquences et 1 091 caractères.

Une séquence consensus a été réalisée pour chacun des alignements et les amorces de séquence SEQ ID NO: 2, 3 et 5 ont été placées pour visualiser la région d'intérêt. Pour chaque région délimitée, les extrémités 5' et 3' ont été rallongées de 50 bp.

Les séquences extraites sont les suivantes :

- la séquence consensus SEQ ID NO: 13 pour *Ostreopsis* cf. *siamensis,* et
- la séquence consensus SEQ ID NO: 14 pour *Ostreopsis* cf. *ovata.*

Ces séquences ont été synthétisées par le service Eurofins Genomics dans un plasmide pEX-A128. La concentration initiale en copies est déterminée à partir de la quantité de plasmides synthétisés avec la formule suivante :

$$\text{Nombre de copies} = (A \times 6{,}022{.}10^{23})/(L \times 10^{9} \times 660)$$

dans laquelle A est la masse de plasmide en ng, L est la longueur (en bases) du plasmide comprenant la séquence consensus, $6{,}022{.}10^{23}$ est le nombre d'Avogadro et 660 est le poids moléculaire moyen d'une paire de base.

Dans notre cas, 1.2 μg de plasmide (A) ont été resuspendus dans 100μl d'eau de grade moléculaire, ce qui donne $3{,}95{.}10^{11}$ copies/100μl (taille du plasmide : 2 450 bp ; taille du fragment : 323 bp) pour *O. cf. siamensis* et $3{,}97{.}10^{11}$ copies/100μl (taille du plasmide : 2 450 bp ; taille du fragment : 311 bp) pour O. cf. *ovata.*

Ces plasmides ont permis d'établir de nouvelles gammes étalons pour chacune des deux espèces (cf. Fig. 6 et 7). Le premier point de chaque gamme est dilué à une concentration de $4{,}0{.}10^{6}$ copies.μl$^{-1}$. Une série de dilutions au 10ème est ensuite réalisée pour obtenir un total de 7 points de référence allant de $4{,}0{.}10^{6}$ à 4 copies.μl$^{-1}$.

## Revendications

1. Méthode de détection d'au moins un microorganisme dans au moins un échantillon, comprenant les étapes suivantes :

   a. filtration dudit échantillon, pour obtenir un rétentat comprenant des microorganismes,
   b. extraction de l'ADN des microorganismes du rétentat, pour obtenir une solution comprenant de l'ADN extrait,
   c. purification de l'ADN de la solution comprenant l'ADN extrait, pour obtenir une solution d'ADN purifié,
   d. amplification de l'ADN de la solution d'ADN purifié avec au moins un couple d'amorces spécifique du microorganisme à détecter, et
   e. détermination de la présence et/ou quantification du microorganisme à détecter dans l'échantillon

   **caractérisée en ce que** ledit au moins un microorganisme à détecter est une microalgue du genre *Ostreopsis* ou une microalgue de l'espèce *Ostreopsis* cf. *ovata* et **en ce que**, dans l'étape d), le couple d'amorces comprend un mélange d'amorces anti-sens de séquence SEQ ID NO: 1.

2. Méthode de détection selon la revendication 1, **caractérisée en ce que** la méthode comprend la détection d'une microalgue de l'espèce *Ostreopsis* cf. *siamensis.*

3. Méthode de détection selon la revendication 1 ou 2, **caractérisée en ce que** l'étape b) d'extraction comprend une étape de lyse chimique, pour obtenir un premier lysat et, optionnellement, une étape de lyse mécanique dudit premier lysat.

4. Méthode de détection selon la revendication 3, **caractérisée en ce que** l'étape de lyse chimique comprend de placer le filtre dans un récipient en présence d'une solution de lyse, de sorte que le filtre et le rétentat présent sur le filtre soient au contact de la solution de lyse, et sous agitation.

5. Méthode de détection selon l'une des revendications 1 à 4, dans laquelle l'étape c) de purification comprend une étape de clarification et une étape de précipitation.

6. Méthode de détection selon l'une des revendications 1 à 5, dans laquelle l'étape d) comprend l'amplification de l'ADN d'au moins deux échantillons de référence, chaque échantillon de référence comprenant une concentration connue d'un plasmide, ledit plasmide comprenant une séquence consensus du microorganisme à détecter.

7. Mélange d'amorces anti-sens approprié pour la détection d'une microalgue du genre *Ostreopsis* et/ou d'une microalgue de l'espèce *Ostreopsis* cf. *ovata,* **caractérisé en ce que** les amorces anti-sens dudit mélange comprennent la séquence SEQ ID NO: 1.

8. Kit approprié pour la détection d'une microalgue du genre *Ostreopsis* et/ou d'une microalgue de l'espèce *Ostreopsis* cf. *ovata,* ledit kit comprenant le mélange d'amorces anti-sens selon la revendication 7.

9. Utilisation du mélange d'amorces anti-sens selon la revendication 7 ou du kit selon la revendication 8, pour la détection d'au moins une microalgue du genre *Ostreopsis* et/ou d'au moins une microalgue de l'espèce *Ostreopsis* cf. *ovata.*

**10.** Plasmide approprié pour la quantification d'une microalgue dans un échantillon, ledit plasmide comprenant la séquence consensus SEQ ID NO: 13 ou SEQ ID NO: 14.

FIG.1

FIG.2

FIG.3

**FIG.4**

FIG.5

FIG.6

EP 4 458 966 A1

y = -3,79x + 40,20
R² = 0,99
E = 83,6 %

Nombre de copies *O. cf. ovata*

FIG.7

**RAPPORT DE RECHERCHE EUROPEENNE**

Numéro de la demande

EP 24 17 4116

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| A | FU ZHENGXU ET AL: "Diversity and distribution of harmful microalgae in the Gulf of Thailand assessed by DNA metabarcoding", HARMFUL ALGAE, ELSEVIER, AMSTERDAM, NL, vol. 106, 1 juin 2021 (2021-06-01), XP086637591, ISSN: 1568-9883, DOI: 10.1016/J.HAL.2021.102063 [extrait le 2021-06-11] * p. 2, colonne de droite, 2ème, 4ème et 5ème para. * ----- | 1-10 | INV. C12N15/10 C12Q1/6895 |
| A | CN 111 607 636 A (UNIV JIANGSU OCEAN) 1 septembre 2020 (2020-09-01) * le document en entier * ----- | 1-10 | |
| A | BURGER STEFAN ET AL: "Labslice XL - A Centrifugal Microfluidic Cartridge for the Automated Bio-Chemical Processing of Industrial Process Water", 2019 20TH INTERNATIONAL CONFERENCE ON SOLID-STATE SENSORS, ACTUATORS AND MICROSYSTEMS & EUROSENSORS XXXIII (TRANSDUCERS & EUROSENSORS XXXIII), IEEE, 23 juin 2019 (2019-06-23), pages 122-125, XP033600209, DOI: 10.1109/TRANSDUCERS.2019.8808601 * p. 122, colonne de gauche, dernier para. - colonne de droite; fig. 1 * ----- | 1-10 | |
| A | WO 2022/229690 A1 (PINDI PAVAN KUMAR [IN]) 3 novembre 2022 (2022-11-03) * para. 43-46 * ----- | 1-10 | |
| | -/-- | | |

DOMAINES TECHNIQUES RECHERCHES (IPC)

C12N
C12Q
C40B

Le présent rapport a été établi pour toutes les revendications

| Lieu de la recherche | Date d'achèvement de la recherche | Examinateur |
|---|---|---|
| Munich | 18 septembre 2024 | Ripaud, Leslie |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

page 1 de 3

Europäisches
Patentamt

European
Patent Office

Office européen
des brevets

# RAPPORT DE RECHERCHE EUROPEENNE

Numéro de la demande

EP 24 17 4116

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X,D | PENNA A. ET AL: "Monitoring of HAB species in the Mediterranean Sea through molecular methods", JOURNAL OF PLANKTON RESEARCH , vol. 29, no. 1 4 octobre 2006 (2006-10-04), pages 19-38, XP093098846, Oxford University Press ISSN: 0142-7873, DOI: 10.1093/plankt/fbl053 Extrait de l'Internet: URL:https://academic.oup.com/plankt/article/29/1/19/2963060 * résumé; p. 23, para. reliant colonnes de gauche et droite; p. 25, colonne de droite, 1er para.; tableau III * | 1-10 | |
| X,D | DROUET KÉVIN ET AL: "Current distribution and potential expansion of the harmful benthic dinoflagellate Ostreopsis cf. siamensis towards the warming waters of the Bay of Biscay, North-East Atlantic", ENVIRONMENTAL MICROBIOLOGY , vol. 23, no. 9 15 février 2021 (2021-02-15), pages 4956-4979, XP093098844, GB ISSN: 1462-2912, DOI: 10.1111/1462-2920.15406 Extrait de l'Internet: URL:https://onlinelibrary.wiley.com/doi/full-xml/10.1111/1462-2920.15406 * p. 4970-4971 * | 1-10 | DOMAINES TECHNIQUES RECHERCHES (IPC) |

-/--

Le présent rapport a été établi pour toutes les revendications

| Lieu de la recherche | Date d'achèvement de la recherche | Examinateur |
|---|---|---|
| Munich | 18 septembre 2024 | Ripaud, Leslie |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
& : membre de la même famille, document correspondant

EPO FORM 1503 03.82 (P04C02)

page 2 de 3

RAPPORT DE RECHERCHE EUROPEENNE

Europäisches Patentamt
European Patent Office
Office européen des brevets

Numéro de la demande

EP 24 17 4116

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| A | CHOMÉRAT NICOLAS ET AL: "First Characterization of Ostreopsis cf. ovata (Dinophyceae) and Detection of Ovatoxins during a Multispecific and Toxic Ostreopsis Bloom on French Atlantic Coast", MARINE DRUGS, vol. 20, no. 7, 18 juillet 2022 (2022-07-18), page 461, XP093100406, DOI: 10.3390/md20070461 * le document en entier * ----- | 1-10 | |
| A | DATABASE EMBL [Online] 11 février 2020 (2020-02-11), "Ostreopsis cf. ovata strain UNR-03 internal transcribed spacer 1, partial sequence; 5.8S ribosomal RNA gene, complete sequence; and internal transcribed spacer 2, partial sequence.", XP093100408, extrait de EBI accession no. EM_STD:MN560105 Database accession no. MN560105 * le document en entier * ----- | 1-10 | DOMAINES TECHNIQUES RECHERCHES (IPC) |

Le présent rapport a été établi pour toutes les revendications

| Lieu de la recherche | Date d'achèvement de la recherche | Examinateur |
|---|---|---|
| Munich | 18 septembre 2024 | Ripaud, Leslie |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
& : membre de la même famille, document correspondant

EPO FORM 1503 03.82 (P04C02)

## ANNEXE AU RAPPORT DE RECHERCHE EUROPEENNE
## RELATIF A LA DEMANDE DE BREVET EUROPEEN NO.

EP 24 17 4116

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

18-09-2024

| Document brevet cité au rapport de recherche | Date de publication | Membre(s) de la famille de brevet(s) | Date de publication |
|---|---|---|---|
| CN 111607636 A | 01-09-2020 | AUCUN | |
| WO 2022229690 A1 | 03-11-2022 | AUCUN | |

Pour tout renseignement concernant cette annexe : voir Journal Officiel de l'Office européen des brevets, No.12/82

# RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **PENNA et al.** *Journal of Plankton Research,* 2007, vol. 29 (1), 19-38

- **DROUET.** *Environmental Microbiology,* 2021, vol. 23, 4956-4979